# EUROPEAN PATENT APPLICATION

(11) **EP 2 499 913 A1**
(43) Date of publication of application: **19.09.2012**
(21) Application number: 11382069.0
(22) Date of filing: 14.03.2011
(51) Int. Cl.: A01N 47/44, A01N 25/00, A61K 31/155, A61K 47/10, A61K 47/20, A61K 9/00, A01P 1/00

(54) **Antiseptic solutions comprising chlorhexidine or its salt and an anionic dye and their preparation**

(71) Applicant: Combino Pharm, S.L., 08970 Sant Joan Despi, Barcelona (ES)
(72) Inventor: Puigvert Colomer, Marina, 08014 Barcelona (ES); Lloret Perez, Sergio, 08030 Barcelona (ES)
(74) Representative: Pons Ariño, Angel

(57) **Abstract**

The present invention relates to a stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 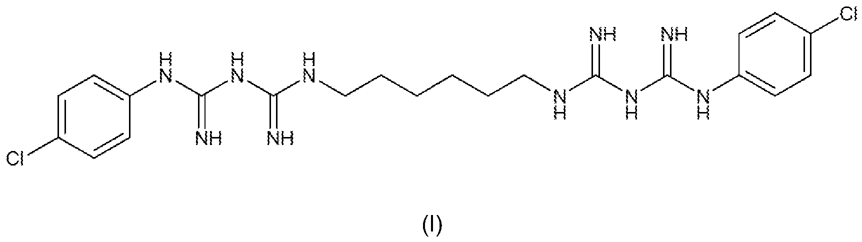 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient.

## Description

The present invention is concerned with an antiseptic solution comprising a di(4-chloro-phenyldiguanido) derivative, and improved processes for obtaining it.

### BACKGROUND OF THE INVENTION

Chlorhexidine is the international common accepted name for N,N"-Bis(4-chlorophenyl)-3,12-diimino-2,4,11,13-tetraazatetradecanediimidamide [also known as 1,6-di(4'-chlorophenyldiguanido)hexane], and has an empirical formula of C22H30Cl2N10 and a molecular weight of 505.45.

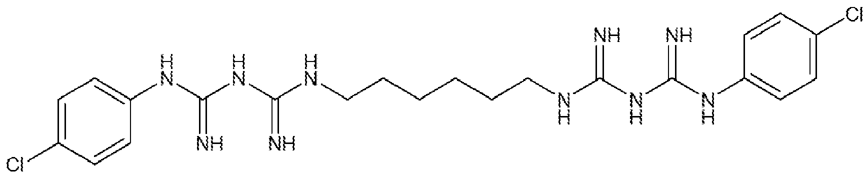

Chlorhexidine is a well-known topical antiseptic and disinfectant that has been used for more than 30 years. Most often chlorhexidine products are used for a number of applications such as daily hygiene application, oral antiseptic applications, hand and skin disinfection, general disinfection (equipment, surfaces and textiles), etc. Chlorhexidine has been shown to be highly and broadly bactericidal with low levels of toxicity and strong skin binding properties. Chlorhexidine is primarly bactericidal against gram-positive and gram-negative, and its activity extends to yeasts, including Candila albicans. The bactericidal activity of chlorhexidine differs from that observed with povidone-iodine or 70% isopropyl alcohol. Chlorhexidine has demonstrated an immediate bactericidal effect as well as a cumulative effect that persists for hours and even days after it is applied and, unlike iodophors, the germicidal activity of chlorhexidine has shown statistically significant efficacy in reducing bacterial sounts in the presence of blood and other protein-rich biomaterials.

Chlorhexidine in solution is a non-colored and clear liquid and, consequently, it is very difficult for the user to see where the liquid has been applied. However, it is not a petty issue, but an important one. In many situations it is highly relevant knowing where exactly the antiseptic, such as a chlorhexidine solution, has been applied. For example, chlorhexidine is often applied in cathethers to desinfect materials and surfaces or patient's skin just prior to surgery. It is essential that health professionals, such as a nurse, surgeon, be able to see where the preoperative liquid has been applied. Therefore, it is desirable to use colored chlorhexidine solution to discern not only that the antiseptic has been applied but also where the liquid has been applied.

However a numerous problems are encountered when colour, such as a tint or dye is added to an antiseptic solution of chlorhexidine in amount sufficient to stain a patient's skin or surgical field before surgery, when applied. The tint or dye reacts easily with chlorhexidine causing it to be to some extent, either precipitated or inactivated. Thus, on one hand, if colorant settles out of the liquid there may be nonuniform distribution of the colored liquid when applied, and on the other hand when it becomes inactivated, the impurity profile increases affecting chemical stability of the product and therefore the shelf life of the liquid may be shortened.

In view of the above discussion, development of colored solutions of chlorhexidine, with a sufficient shelf life has proved to be difficult. Additionally the development of colored solutions of chlorhexidine, advantageously prepared by efficient formulation processes has proved problematic.

US 2004/017989 acknowledges the above discussed problem associated with colored chlorhexidine solutions. US 2004/017989 describes that the problem can be solved by use of specific, specialized applicator for applying chlorhexidine solution to a surface which comprises at least one ampoule formed of a frangible material and adapted to contain liquid to be applied; at least one hollow body defining an internal chamber adapted to receive at least one ampoule; and at least one porous element that contains colorant, wherein the porous element is positioned such that liquid flows through the porous element when at least one ampoule is fractured and colorant is transferred to the liquid to be applied.

However it should be noted that the use of specific applicators to administer antiseptic solution or its previous reconstitution prior use, in many cases may result inconvenient or even inapplicable for some applications.

WO 2007/130981 discloses solution of chlorhexidine together with cationic dye in an amount sufficient to stain patient's skin. It is believed that as chlorhexidine pharmaceutically acceptable salts are cationic compounds, as such, are compatible with other cationic and non ionic substances, but are chemically incompatible with anionic compounds.

However it should be noted that numerous disadvantages are related with use of cationic dyes in antiseptic formulation, generally arised from adverse toxicological and cancerogenic effects noticed. Concerns over the safety of use of cationic dyes in products like antiseptic solutions are associated with reports of hypersensitivity and hyperkinetic activity, especially among children. Thus, cationic dyes can be hazard to health and products containing it.

Moreover the PCT application WO 2007/130981 teaches that an anionic dye may be used provided that the antiseptic solution also comprises a cationic excipient. Said application teaches that anionic dyes, including FD&C dyes, form a precipitant with chlorhexidine, even at very low concentrations. As such, adding an anionic dye alone to an aqueous chlorhexidine solution would remove a significant fraction of the chlorhexidine from solution, decreasing the efficacy of the solution. The precipitant would be formed by an insoluble salt of a chlorhexidine cation and at least one dye anion.

WO 2007/130982 tries to solve the above described problems by providing the solutions of chlorhexidine together with an anionic dye and a cationic excipient. WO 2007/130982 describes that the use of a cationic excipient together with an anionic excipient is essential and that as the negative charge of an anionic dye is "hidden" from the chlorhexidine by a cationic excipient, the chlorhexidine-dye salt will not immediately form.

However, it should be noted that the use of additional excipients in antiseptic solution, especially cationic excipeints that mainly include as described in WO 2007/130982: cationic detergents, surfactants or excipeints containing quaternary nitrogen; may cause dangerous adverse affects related with allergy, intolerance and/or irritation.

In addition, according to the Summary of Product Characteristics for ChloraPrep® 2% w/v / 70% v/v cutaneous solution, chlorhexidine is incompatible with anionic agents (http://www.medicines.org.uk/emc/medicine/22302/SPC/chloraprep/).

As referred to above, chlorhexidine is known to be unstable in colored solution. This instability can be problematic in the preparation and storage of liquid. There is a need, therefore, to provide colored antiseptic solutions comprising chlorhexidine which are safe to use, have improved properties and good chemical stability. There is also a need for colored antiseptic chlorhexidine solution which can advantageously be prepared by efficient formulation processes. Such compositions, including colored solutions, comprising chlorhexidine, and formulation processes therefore, are now provided by the present invention substantially as hereinafter described in greater detail.

Other key issue when developing pharmaceutical compositions is the possible hypersensitivity of the patient whom the antiseptic is going to be applied. When a patient is hypersensible to any of the excipients entails that the pharmaceutical drug can not be used to such patient, although the patient fully tolerates to the mains pharmaceutical active ingredients.

### DETAILED DESCRIPTION OF THE INVENTION

The object of the present invention is the provision of an antiseptic solution comprising the di(4-chloro-phenyldiguanido) derivative of formula (I), or its pharmaceutically acceptable salt, which shows an excellent compatibility with anionic dyes and, even without the presence of any cationic excipient.

An antiseptic solution as provided by the present invention can offer various advantages. For example, the present invention can provide a solution comprising the di(4-chloro-phenyldiguanido) derivative of formula (I), or its pharmaceutically acceptable salt, and an anionic dye in an amount sufficient to stain a patient's skin or surgical field before surgery, in the absence of additional excipients, apart from the solvent.

The antiseptic solutions of the invention have a rapid onset and long lasting activity against potential pathogens. The antiseptic solutions of the inventions show a long shelf life, even, in some cases, longer than 18 months.

The medicinal product is to be used for disinfection of the skin or surgical field prior to invasive medical procedures

A further advantage of the invention relates to the fact that the antiseptic solutions of the invention are not contra-indicated where patients have shown previous hypersensitivity to cationic excipients or cationic dyes

The antiseptic solutions of the invention have good pharmacokinetic properties. For example, the absence of the cationic dye reduces the absorption of isopropyl alcohol or of chlorhexidine through intact skin. Since there is little percutaneous absorption of the antiseptic solutions of the invention they are indicated, for example, for use on pre-injection sites, pharmacodynamic studies have not been undertaken

Without being bound by the theory, the antiseptic solutions of the invention exert their lethal effect upon bacterial cells through nonspecific interaction with acidic phospholipids of the cell membranes. The antiseptic solutions of the invention are an excellent both antiseptic and disinfectant and they are bactericidal or bacteriostatic against a wide range of gram-positive and gram-negative bacteria. It is more effective against Gram-positive than Gram-negative bacteria, and some species of Pseudomonas and Proteus have low susceptibility. It inhibits some viruses and is active against some fungi. It is inactive against bacterial spores at room temperature. Combinations in alcoholic solution, preferably isopropyl alcohol and water, are used to enhance efficacy.

It was surprisingly found that the addition of specific amount of an anionic dye into the antiseptic solution of the present invention can prevent precipitation and can assure chemical stability and shelf life of the product. The antiseptic solution of the present invention are stable in relative extreme hot or cold temperature conditions. Specifically the present invention relates to a stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 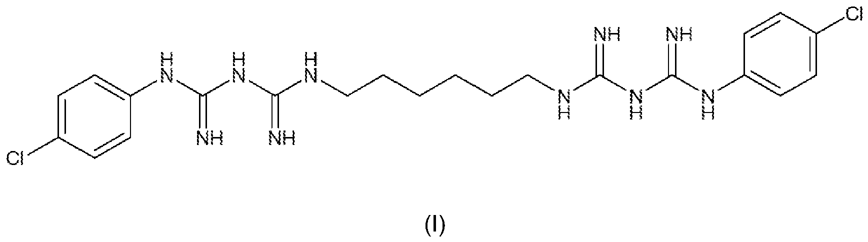 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient.

In the context of the present invention the term "cationic excipient" refers to the cationic excipient as disclosed in the WO2007/130982, herein included by reference. Cationic dyes are also considered as cationic excipients in the context of the present invention.

In the context of present invention the term "antiseptic solution" refers to a any liquid product which comprises said di(4-chloro-phenyldiguanido) derivative of formula (I) or a pharmaceutically acceptable salt thereof where all the components of the product are completely dissolved and the said solution is ready for administration and does not require for its application neither any previous preparation, like reconstitution, nor any specific, specialised applicators. The antiseptic solution of the invention as used here means the antibacterial, disinfectant solution for use for skin disinfection, handwashing, oral care, irrigation of surgical wounds, the urinary bladder or vagina, topical treatment of burn wounds, for treatment of peritonitis in peritoneal dialysis for all the purposes related to its antiseptic, disinfectant properties.

The term "essentially free" refers to an insubstantial amount, for example of cationic excipient. In a preferred embodiment, substantially or essentially free refers to less than about 0.01 weight percent, preferably less than about 0.001 weight percent. In the most preferred embodiment the antiseptic solution does not comprise any cationic excipient. Preferably, the antiseptic solution is free of any cationic excipient.

As used herein, the term "stable" refers to a solution that after 24 hours is clear and leaves no residue solid visible to the human eye. Moreover, the stable antiseptic solutions of the invention do not leave residue solids visible to the human eye after a stability test in the following conditions: 25 ± 2°C & 60% ± 5% relative humidity during 3 months. The stable antiseptic solutions of the invention also contain insubstantially amount of p-Chloroaniline impurity, preferably less than 0.25% w/w, after suffering the stability test above mentioned. The stability studies should be carried out as is defined in ICH Topic Q1A "Stability Testing of New Drug Substances and Products" (CPMP/ICH/2736/99).

Although colorants have been added to di(4-chloro-phenyldiguanido) derivative of formula (I) solutions in some applications, a numerous problems are encountered with increased amount of colorants, and non of these applications has suggested the addition of anionic dye in amount sufficient to stain or color, without need of further additional excipeints to obtain the solution which does not precipitate and is chemically stable during shelf life.

Anionic dye here means any colored substance containing auxochromes and thus capable of coloring substances to which it is applied; used for staining and coloring, as a test reagent, and as a therapeutic agent. Anionic dyes are characterized that they have a negative charge and attach to cationic surfaces. Anionic dyes include many compounds from the most varied classes of dyes, which exhibit characteristic differences in structure (e.g., azoic, anthraquinone, triphenylmethane and nitro dyes) but posses as a common feature water-solubilizing, ionic substituents.

An antiseptic solution comprising di(4-chloro-phenyldiguanido) derivative of formula (I) or its pharmaceutically acceptable salt as provided by the present invention comprises an anionic dye in an amount sufficient to stain a patient's skin or surgical field before surgery, when applied. The anionic dyes that may be employed within aqueous antiseptic solutions of the present invention include, but are not limited to, FD&C dyes, such as for example, FD&C Blue No. 1 (Brilliant Blue FCF), FD&C Blue No. 2 (Indigo Carmine), FD&C Green No.3 (Fast Green FCF), FD&C Red No.3 (Erythrosine), FD&C Red No. 40 (Allura Red), Food Red 3 Carmoisine, FD&C Yellow No.5 (Tartrazine), and FD&C Yellow No. 6 (Sunset Yellow FCF). Preferably the antiseptic solution of the present invention comprises FD&C Red No. 40 (Allura Red) anionic dye. In a preferred embodiment the antiseptic solution of any one of the preceding claims, wherein the anionic dye is FD&C Blue No.1, FD&C Blue No.2, FD&C Green No.3, FD&C Red 40, Food Red 3 Carmoisine FD&C Yellow No.5, and FD&C Yellow No.6. Preferably the antiseptic solution of the present invention comprises FD&C Red No. 40 (Allura Red) anionic dye. The such dyes are also known as:
FD&C Blue No.1: CAS 3844-45-9; Blue FCF, Acid Blue 9,Alzen Food Blue No. 1Atracid Blue FG, Erioglaucine, Eriosky blue, Patent Blue AR, Xylene Blue VSG
FD&C Blue No.2: CAS 860-22-0; Indigo carmine, 3,3'-dioxo-2,2'-bis-indolyden-5,5'-disulfonic acid disodium salt, indigotine; 5,5'-indigodisulfonic acid sodium salt
FD&C Green No.3: CAS 2353-45-9; Fast Green FCF, Food green 3, Green 1724, Solid Green FCF, C.I. 42053, ethyl - [4 - [ [4 - [ethyl -[(3 - sulfophenyl) methyl] amino] phenyl] - (4 - hydroxy - 2 - sulfophenyl) methylidene] - 1 - cyclohexa - 2, 5 - dienylidene] - [(3 - sulfophenyl) methyl] azanium
FD&C Red No.3: CAS 16423-68-0; erythrosine, 2-(6-hydroxy-2,4,5,7-tetraiodo-3-oxo-xanthen-9-yl)benzoic acid
FD&C Red No. 40: CAS 25956-17-6, Allura Red, Food Red 17, C.I. 16035, E129, 2-naphthalenesulfonic acid disodium salt, disodium 6-hydroxy-5-((2-methoxy-5-methyl-4-sulfophenyl)azo)-2-naphthalenesulfonate
Food Red 3: CAS 3567-69-9; azorubine, carmoisine, disodium 4-hydroxy-2-[(E)-(4-sulfonato-1-naphthyl)diazenyl]naphthalene-1-sulfonate
FD&C Yellow No.5: CAS 1934-21-0; Tartrazine, Trisodium (4E)-5-oxo-1-(4-sulfonatophenyl)-4-[(4-sulfonatophenyl)hydrazono]-3-pyrazolecarboxylate
FD&C Yellow No.6: CAS 2783-94-0; Orange Yellow S; FD&C Yellow 6; C.I. 15985; E110, Disodium 6-hydroxy-5-[(4-sulfophenyl)azo]-2-naphthalenesulfononate

In a preferred embodiment, therefore, the present invention further provides the specific concentration of anionic dye sufficient to stain a patient's skin or surgical field before surgery, which is normally less than a 5% w/v. Preferably, the concentration of anionic dye may range from about 0.05 % to about 1.0% w/v. More preferably the concentration of anionic dye is within the range of about 0.10% to about 0.60% w/v. Most preferably the concentration of anionic dye is 0.2% w/v. Preferably, the antiseptic solution of the present invention comprises only one anionic dye.

Due to the poor solubility of the di(4-chloro-phenyldiguanido) derivative of formula (I), in a preferred embodiment of the present invention the antiseptic solution of the invention comprises at least one pharmaceutically acceptable salt of di(4-chloro-phenyldiguanido) derivative of formula (I). In a preferred embodiment, the antiseptic solution of the present invention comprises at least one pharmaceutically acceptable salt of di(4-chloro-phenyldiguanido) derivative of formula (I) selected of the group of pharmaceutically acceptable consisting of gluconate, acetate, chloride, bromide, nitrate, sulphate, carbonate and phospanilate.

The concentration of di(4-chloro-phenyldiguanido) derivative of formula (I) or its pharmaceutically acceptable salt in the antiseptic solution of the invention may vary within various embodiments of the present invention. However in preferred embodiment, the concentration of di(4-chloro-phenyldiguanido) derivative of formula (I) or its pharmaceutically acceptable salt is about 0.1 % w/v to 10% w/v. Preferably, the concentration of the antiseptic solution of the present invention is about 0.5% to 4% w/v of the di(4-chloro-phenyldiguanido) derivative of formula (I) or pharmaceutically acceptable salt thereof. Even more preferably, the the antiseptic solution of the present invention comprises about 2% w/v of the di(4-chloro-phenyldiguanido) derivative of formula (I) or pharmaceutically acceptable salt thereof.

The suitable solvent as used to form the antiseptic solution of the invention is a fluid that is compatible with the other ingredients of the composition and is non-toxic when applied to human or animal skin. Suitable solvents include, but are not limited to water, alcohols, acetone, esters, chlorinated hydrocarbons, chlorofluorohydrocarbons. Preferably the solvent of the solution is formed by a suitable solvent, wherein the suitable solvent comprises a mixture of at least one lower alkanol and deionized water. Preferred embodiments include suitable solvents which are solutions formed by water and about 20 to about 95% v/v of at least one C1-C8 lower alkanol like isopropanol, ethanol and other alcohols. Preferably, embodiments include suitable solvents which are solutions formed by water and about 50 to about 90% v/v of at least one C1-C8 lower alkanol. The preferred lower alkanol is isopropanol. In a preferred embodiment, the suitable solvent of the antiseptic solution of the invention consists essentially of water and about 70% v/v of isopropyl alcohol. Preferably the antiseptic solution of the invention is the aqueous lower alkanol solution that consists of deionized water and 70% v/v of isopropyl alcohol.

The terms "lower alcohol" or "lower alkanol" refer to straight chain or branched alkyl residues containing 1 to 8 carbon atoms with at least one hydroxy group, such as methanol, ethanol, n-propanol, isopropanol, n-butanol, 1 -butanol, 2-butanol, tert-butanol and the like

Precipitation here means the formation of any solids in the antiseptic solution of the invention. Precipitation here means the particulate contamination that consists of extraneous, mobile, undissolved particles, other than gas bubbles, unintentionally present in the solution. Precipitation, particulate contamination can be measured by a tests that are intended to provide a simple procedure for the visual assessment of the quality of the antiseptic solution comprising di(4-chloro-phenyldiguanido) derivative of formula (I) or pharmaceutically acceptable salt thereof and an anionic dye in an amount sufficient to stain a patient's skin or surgical field before surgery, when applied, as regards visible particles. One can measure precipitation, particulate contamination of the antiseptic solution of the invention by first removing any adherent labels from the container and washing and drying outside if the container is transparent. If the container is not transparent the antiseptic solution should be placed in previously washed and dried transparent container. Further the container should be gently swirled or inverted ensuring that air bubbles are not introduced, and should be observed. No particulate contamination should be observed. Other validated methods should not be excluded in any way. The chemical stability during shelf life means here a desirable impurity profile that can in particular be associated with an antiseptic solution comprising di(4-chloro-phenyldiguanido) derivative of formula (I) or pharmaceutically acceptable salt thereof and an anionic dye in an amount sufficient to stain a patient's skin or surgical field before surgery, when applied. and can be further illustrated by accompanying. Table 1, where requirement of low levels of p-chloroaniline as an impurity on storage at both room temperature and at 40°C remains on stable level as required during at least 6 months are stated. A method to measure p-chloroaniline impurity is an HPLC method, based on USP33 references and ICH guidelines requirements.

The requirements for the precipitation, particulate formation named as appearance and chemical stability defined by the content of p-chloroaniline are represented by Table 1.

| Test | Requirement |
|---|---|
| Appearance | Coloured solution free of particles |
| p-Chloroanilinae (%) | Not More Than 0.25% |

Table 1. Requirements regarding precipitation, particulate formation and chemical stability of the antiseptic solution comprising di(4-chloro-phenyldiguanido) derivative of formula (I) or pharmaceutically acceptable salt thereof and an anionic dye in an amount sufficient to stain a patient's skin or surgical field before surgery, when applied.

Alternatively, the present invention can provide an antiseptic solution consisting of a pharmaceutically acceptable salt of di(4-chloro-phenyldiguanido) derivative of formula (I) and an anionic dye in an amount sufficient to stain a patient's skin or surgical field before surgery, wherein the solution is an aqueous lower alcohol solution.

Preferred antiseptic solution consists of di(4-chloro-phenyldiguanido) derivative of formula (I) or pharmaceutically acceptable salt thereof, isopropanol, water and an anionic dye in an amount sufficient to stain a patient's skin or surgical field before surgery, when applied

A further particularly preferred antiseptic solution consists of 2% w/v of di(4-chloro-phenyldiguanido) derivative of formula (I) digluconate, 70% v/v of isopropanol, water and an anionic dye in an amount sufficient to stain a patient's skin or surgical field before surgery, when applied.

In a particular preferred embodiment, the invention relates to a stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 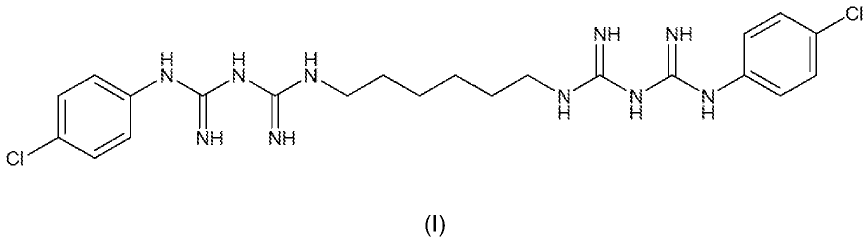 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient, wherein the concentration of the anionic dye is less than 5%, the pharmaceutically acceptable salt thereof of the compound of formula (I) is ranging from 0.1 to 10 % w/v, wherein the solvent of the solution is formed by a suitable solvent, wherein the suitable solvent comprises a mixture of at least one lower alkanol and deionized water.

The present invention still further provides processes of preparing an antiseptic solution according to the present invention as hereinbefore described.

The stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 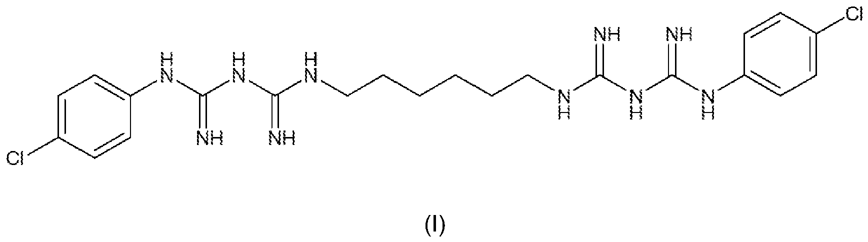 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient of any one of the preceding claims, are prepared by a process comprising at least the following steps: of mixing until dissolution, first at least one anionic dye with the part of the amount of solvent; after mixing until dissolution at least one anionic dye with the part of the amount of solvent, adding the di(4-chloro-phenyldiguanido) derivative of formula (I) or the pharmaceutically acceptable salt thereof and mixing until dissolution; and finally adding the rest of the amount of the specific solvent to the solution formed after dissolving the di(4-chloro-phenyldiguanido) derivative of formula (I) or the pharmaceutically acceptable salt thereof.

It was found that when antiseptic solution of the present invention was prepared by adding all the components in specific order, no precipitation occurred comparing to the process where the specific order was not followed. Surprisingly, the stability of the final solution depends on the order in which the excipients are mixed.

Typically, a process of preparing an antiseptic solution of the present invention comprises the following steps: mixing until complete dissolution, first an anionic dye with the part of the amount of suitable solvent, then adding di(4-chloro-phenyldiguanido) derivative of formula (I) or pharmaceutically acceptable salt thereof previously dissolved in a suitable solvent and mixing until complete dissolution, and finally adding the rest of the amount of the specific solvent.

It is preferred that a process according to the present invention comprises the following steps: mixing until complete dissolution: an anionic dye in deionized water until complete dissolution, then adding a part of the amount of suitable C2-C8 lower alkanol, preferably isopropanol, then adding di(4-chloro-phenyldiguanido) derivative of formula (I) or a pharmaceutically acceptable salt thereof previously dissolved in water and mixing until complete dissolution, and finaly adding the rest of the amount of the C2-C8 lower alkanol, preferably isopropanol.

Without being bound by the theory, it seems that a phenomenon of attraction and association of molecules of anionic dyes and di(4-chloro-phenyldiguanido) derivative of formula (I) or its pharmaceutically acceptable salt by solvents occurs. It seems that, when anionic dye dissolves in water it spreads out and becomes surrounded by C2-C8 lower alkanol, preferably isopropanol molecules. It forms a complex formed by solvent molecules. Then, di(4-chloro-phenyldiguanido) derivative of formula (I) or its pharmaceutically acceptable salt when previously dissolved in water is added into the complex of anionic dye dissolved in water surrounded by C2-C8 lower alkanol, preferably isopropanol, and it forms another complex when interacting with isopropanol of the previous complex. The complexation that is formed, between both systems due to the different polarity of the solvents used, achieves the stability state which assures the antiseptic solution of the present invention being physicochemically stable and with absence of any precipitants/formed particles.

It is further admisible that a process of preparing an antiseptic solution of the present invention might be carried out in the presence of an inert atmosphere so as to desirably facilitate the provision of an antiseptic solution of the present invention being chemically stable.

There is also provided by the present invention a stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 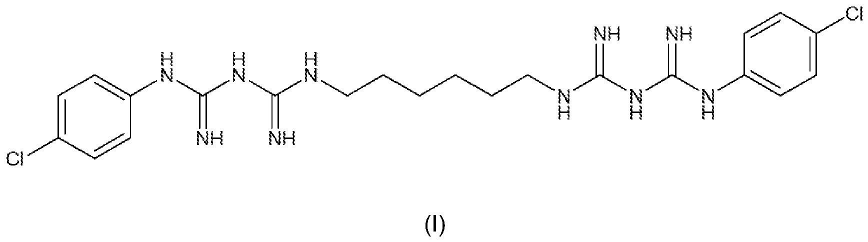 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient of any one of the preceding claims, produced by the process comprising at least the following steps: of mixing until dissolution, first at least one anionic dye with the part of the amount of solvent; after mixing until dissolution at least one anionic dye with the part of the amount of solvent, adding the di(4-chloro-phenyldiguanido) derivative of formula (I) or the pharmaceutically acceptable salt thereof and mixing until dissolution; and finally adding the rest of the amount of the specific solvent to the solution formed after dissolving the di(4-chloro-phenyldiguanido) derivative of formula (I) or the pharmaceutically acceptable salt thereof.

In a preferred embodiment the antiseptic solution comprising di(4-chloro-phenyldiguanido) derivative of formula (I) or pharmaceutically acceptable salt thereof and an anionic dye in an amount sufficient to stain a patient's skin or surgical field before surgery, when applied, is obtainable by the process that comprises the following steps: mixing until complete dissolution, first an anionic dye in water until complete dissolution, then adding a part of the amount of suitable C2-C8 lower alkanol, preferably isopropanol, then adding di(4-chloro-phenyldiguanido) derivative of formula (I) or pharmaceutically acceptable salt thereof and mixing until complete dissolution, and finally adding the rest of the amount of the C2-C8 lower alkanol, preferably isopropanol.

In a further particularly preferred embodiment the antiseptic solution comprising di(4-chloro-phenyldiguanido) derivative of formula (I) digluconate thereof and FD&C Red No. 40 (Allura Red) anionic dye in an amount sufficient to stain a patient's skin or surgical field before surgery, when applied, is obtainable by the process that comprises the following steps: mixing until complete dissolution, first FD&C Red No. 40 (Allura Red) anionic dye in water until complete dissolution, then adding 80% of the amount of isopropanol, then adding di(4-chloro-phenyldiguanido) derivative of formula (I) digluconate and mixing until complete dissolution, and finaly adding the rest of the amount of isopropanol.

The di(4-chloro-phenyldiguanido) derivative of formula (I) is also known as chlorhexidine.

An antiseptic solution as provided in accordance with the present invention are suitable for use for skin disinfection, handwashing, oral care, irrigation of surgical wounds, the urinary bladder or vagina, topical treatment of burn wounds, for treatment of peritonitis in peritoneal dialysis for all the purposes related to its antiseptic, disinfectant properties to prevent infections such a nocosomial infection, surgical-site infections, catheter-related infections, surgical wound infections, oral infections.

The term "about" when used in the present invention preceding a number and referring to it, is meant to designate any value which lies within the range defined by the number ±10% of its value, preferably a range defined by the number ±5%, more preferably range defined by the number ±2%, still more preferably a range defined by the number ±1%. For example "about 10" should be construed as meaning within the range of 9 to 11, preferably within the range of 9.5 to 10.5, more preferably within the range of 9.8 to 10.2, and still more preferably within the range of 9.9 to 10.1.

### Examples

The present invention will now be further illustrated by reference to the following Examples, which do not limit the scope of the invention in any way.

### Example 1:

| Formulation 1: | |
|---|---|
| Component | |
| Chlorhexidine digluconate | 2% w/v |
| Anionic dye FD&C Red No. 40 (Allura Red) | 0.2% w/v |
| Isopropyl alcohol | 70% v/v |
| Deionizied water | qsp 100 ml |

| Formulation 2: | |
|---|---|
| Component | |
| Chlorhexidine digluconate | 2% w/v |
| Anionic dye FD&C Red No. 40 (Allura Red) | 0.45% w/v |
| Isopropyl alcohol | 70% v/v |
| Deionizied water | qsp 100 ml |

| Formulation 3: | |
|---|---|
| Component | |
| Chlorhexidine digluconate | 2% w/v |
| Anionic dye FD&C Red No. 40 (Allura Red) | 0.2% w/v |
| Deionizied water | qsp 100 ml |
| Ethyl alcohol 99,9% | 70% v/v |

| Formulation 4: | |
|---|---|
| Component | |
| Chlorhexidine digluconate 20% v/w aqueous solution | 1 ml |
| Anionic dye Food Red 3 Carmoisina | 0.07% w/v |
| Ethyl alcohol 99,9% | qsp 100 ml |

### Process:

Anionic dye FD&C Red No. 40 (Allura Red) was dissolved in deionized water (Formulation 1, 2 and 3) and anionic dye Food Red 3 Carmoisine was dissolved in ethanol (Formulation 4). The mixture was stirred with a magnetic stirrer until dissolution was complete at room temperature. 80% of theoretical weight of isopropanol (Formulation 1 and 2) and ethanol (Formulation 3 and 4) was added to the above solution and stirred with magnetic stirrer until homogenity complete for 10 minutes at room temperature. Di(4-chloro-phenyldiguanido) derivative of formula (I) digluconate 20% v/w aqueous solution was weighed and added to the above solution. The mixture was stirred with magnetic stirrer until complete homogeneity for 10 minutes at room temperature. The remaining part of isopropanol (Formulation 1 and 2) and ethanol (Formulation 3 and 4) was added, and the mixture was stirred with magnetic stirrer for another 10 minutes at room temperature. The pH and density of the resulting solution were measured. The solution was filtered through clarificant filter of 100µm filters and filled in plastic HDPE (High Density Polyethylele) vials.

### Example 2:

Stability tests were performed for the Formulations 1, 2 and 3 of the above example, as represented by Table 2 and Table 3.

**Table 2. Stability test condition: 25 ± 2°C & 60% ± 5% relative humidity**

| | Appearance | | | p-Chloroaniline | | |
|---|---|---|---|---|---|---|
| | [Coloured solution free of particles] | | | [Not More Than 0.25%] | | |
| | | | | | | |
| | 0 | 3months | 6months | 0 | 3months | 6months |
| Formulation 1 | Conforms | Conforms | Conforms | 0.01% | 0.02% | 0.02% |
| Formulation 2 | Conforms | Conforms | Conforms | 0.01% | 0.01 % | 0.02% |
| Formulation 3 | Conforms | Conforms | Conforms | 0.02% | 0.04% | Not tested |
| Formulation 4 | Conforms | Conforms | Conforms | 0.02% | Not tested | 0.06% |

**Table 3. Stability test condition: 40 ± 2°C & 75% ± 5% relative humidity**

| | Appearance | | | p-Chloroaniline | | |
|---|---|---|---|---|---|---|
| | [Coloured solution free of particles] | | | [Not More Than 0.25%] | | |
| | | | | | | |
| | 0 | 3months | 6months | 0 | 3months | 6months |
| Formulation 1 | Conforms | Conforms | Conforms | 0.01 % | 0.06% | 0.14% |
| Formulation 2 | Conforms | Conforms | Conforms | 0.01 % | 0.08% | 0.12% |

## Claims

1. A stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 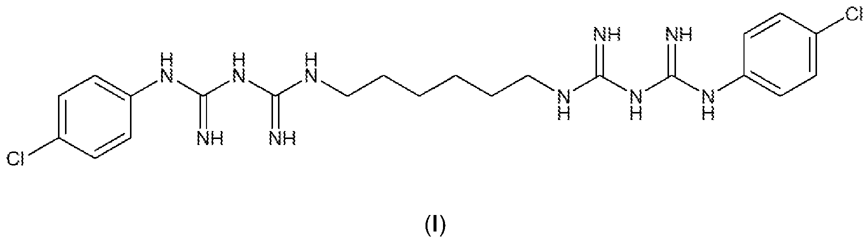 and at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient.

2. The antiseptic solution of claim 1, wherein the antiseptic solution is free of any cationic excipient.

3. The antiseptic solution of any one of the preceding claims, wherein the anionic dye is FD&C Blue No.1, FD&C Blue No.2, FD&C Green No.3" FD&C Red 40, Food Red 4 Carmoisine, FD&C Yellow No.5, and FD&C Yellow No.6.

4. The antiseptic solution of any one of the preceding claims, wherein the concentration of the anionic dye is less than 5%, preferably ranging from about 0.05 % w/v to about 1.0% w/v, more preferably ranging from about 0.10% w/v to about 0.60% w/v, even more preferably is about 0,2% w/v.

5. The antiseptic solution of any one of the preceding claims, comprising at least one pharmaceutically acceptable salt of di(4-chloro-phenyldiguanido) derivative of formula (I).

6. The antiseptic solution of the preceding claim, comprising at least one pharmaceutically acceptable salt of di(4-chloro-phenyldiguanido) derivative of formula (I) selected of the group of pharmaceutically acceptable consisting of gluconate, acetate, chloride, bromide, nitrate, sulphate, carbonate and phospanilate.

7. The antiseptic solution of any one of the preceding claims, wherein the concentration of di(4-chloro-phenyldiguanido) derivative of formula (I) or the pharmaceutically acceptable salt thereof is ranging from 0.1 to 10 % w/v, preferably from 0.5 to 4% w/v, even more preferably about 2% w/v.

8. The antiseptic solution of any one of preceding claims, wherein the solvent of the solution is formed by a suitable solvent, wherein the suitable solvent comprises a mixture of at least one lower alkanol and deionized water.

9. The antiseptic solution of any one of preceding claims, wherein the solvent of the solution is formed by a suitable solvent, wherein the suitable solvent comprises a mixture formed by water and about 20 to about 95% v/v of at least one lower alkanol, preferably, formed by water and about 50 to about 90% v/v of at least one lower alkanol, and more preferably formed by water and about 70% v/v of at least one lower alkanol.

10. The antiseptic solution of any of the two preceding claims, wherein the lower alkanol is isopropanol.

11. The antiseptic solution of any one of the three preceding claims, wherein the suitable solvent consists essentially of water and about 70% v/v of isopropyl alcohol.

12. A process of preparing a stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 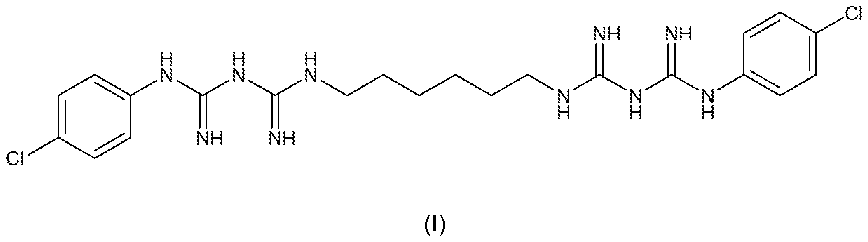 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient of any one of the preceding claims, wherein the process comprising at least the following steps: of mixing until dissolution, first at least one anionic dye with the part of the amount of solvent; after mixing until dissolution at least one anionic dye with the part of the amount of solvent, adding the di(4-chloro-phenyldiguanido) derivative of formula (I) or the pharmaceutically acceptable salt thereof and mixing until dissolution; and finally adding the rest of the amount of the specific solvent to the solution formed after dissolving the di(4-chloro-phenyldiguanido) derivative of formula (I) or the pharmaceutically acceptable salt thereof.

13. A stable antiseptic solution comprising: at least one di(4-chloro-phenyldiguanido) derivative or a pharmaceutically acceptable salt thereof, wherein said di(4-chloro-phenyldiguanido) derivative or a pharmaceutically acceptable salt thereof is a compound of formula (I) or a pharmaceutically acceptable salt thereof; 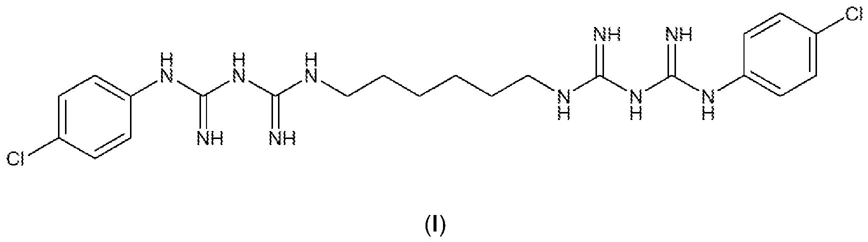 and an at least one anionic dye, wherein the anionic dye or the total amount of anionic dyes present in the antiseptic solution are in an amount sufficient to stain a patient's skin or surgical field before surgery, when applied; wherein the antiseptic solution is essentially free of any cationic excipient of any one of the preceding claims, produced by the process comprising at least the following steps: of mixing until dissolution, first at least one anionic dye with the part of the amount of solvent; after mixing until dissolution at least one anionic dye with the part of the amount of solvent, adding the di(4-chloro-phenyldiguanido) derivative of formula (I) or the pharmaceutically acceptable salt thereof and mixing until dissolution; and finally adding the rest of the amount of the specific solvent to the solution formed after dissolving the di(4-chloro-phenyldiguanido) derivative of formula (I) or the pharmaceutically acceptable salt thereof.
